# EUROPEAN PATENT SPECIFICATION

(11) **EP 2 341 832 B1**
(45) Date of publication and mention of the grant of the patent: **16.07.2014**
(21) Application number: 09741025.2
(22) Date of filing: 07.10.2009
(51) Int. Cl.: A61B 6/00, A61B 6/04, A61B 8/08

(54) **MAMMOGRAPHY-APPARATUS AND METHOD FOR SCREENING THE OCCURRENCE OF MALIGNANT CELLS**
MAMMOGRAPHIEGERÄT UND VERFAHREN ZUR UNTERSUCHUNG DES AUFTRETENS VON MALIGNEN ZELLEN
APPAREIL DE MAMMOGRAPHIE ET PROCÉDÉ DE DÉPISTAGE DE L'OCCURRENCE DE CELLULES MALIGNES

(30) Priority: 13.10.2008 NL 2002092; 03.07.2009 NL 2003122
(43) Date of publication of application: 13.07.2011
(73) Proprietor: Academisch Medisch Centrum bij de Universiteit van Amsterdam, 1105 AZ Amsterdam (NL)
(72) Inventor: DEN HEETEN, Gerhard Johan, NL-1019 GM Amsterdam (NL); GRIMBERGEN, Cornelis Antonius, NL-1391 AT Abcoude (NL)
(74) Representative: Van Breda, Jacobus
(86) International application number: PCT/NL2009/050601
(87) International publication number: WO 2010/044660

(56) References cited:
- EP-A- 1 493 380
- WO-A1-01/17424
- WO-A1-97/27801
- US-A1- 2006 262 903
- US-A1- 2008 249 415
- US-B1- 6 694 173
- VAARTJES S E; VAN HESPEN J C G; KLAASE J M; VAN DEN ENGH F M; THE A K H; STEENBERGEN W; VAN LEEUWEN T G; MANOHAR S: "First clinical trials of the Twente photoacoustic mammoscope (PAM)" CONFERENCE PAPER; JOURNAL PAPER; PROCEEDINGS OF THE SPIE - THE INTERNATIONAL SOCIETY FOR OPTICAL ENGINEERING , ISSN 0277-786X, vol. 6629, 2007, pages 1-12, XP002513416 USA
- KHAMAPIRAD T ET AL: "Diagnostic imaging of breast cancer with LOIS: clinical feasibility" PROCEEDINGS OF THE SPIE - THE INTERNATIONAL SOCIETY FOR OPTICAL ENGINEERING SPIE-INT. SOC. OPT. ENG USA, vol. 5697, no. 1, 2005, pages 35-44, XP002513417 ISSN: 0277-786X

## Description

The invention primarily relates to a mammography-apparatus for detecting malignant cells in a breast comprising an X-ray source, an X-ray detector and a paddle for pressing the breast against said X-ray detector.

Such a mammography-apparatus is commonly known for use in a method for early screening the occurrence of malignant cells in a breast.

To date the lemma "mammography" on Wikipedia reads that in many countries routine mammography of older women is encouraged as a screening method to diagnose early breast cancer and that at this time mammography alone with physical breast examination is the modality of choice for screening such early breast cancer. Ultrasound ductography and magnetic resonance imaging are adjuncts to mammography, whereby ultrasound is typically used for further evaluation of masses found on mammography or palpable masses not seen on mammograms. Mammography as such is reported to have a false negative (missed cancer) rate (depending on the investigated population) of at least 20%. X-ray imaging is known to have a poor discriminatory nature when comparing malignant cells with the surrounding cells of normal glandular tissue. It is further known that invasive breast tumours in more dense breasts (which are overrepresented in lower age groups, yet also occur with older women) are difficult to depict with X-ray imaging. They can easily hide themselves in normal glandular tissue. The main difference on a microscopic level is that these invasive tumours exhibit a phenomenon called neo-angiogenesis: in these tumours a large amount of newly formed tortuous vessels can be seen with haemoglobin containing blood cells, necessary to feed the (fast growing) tumour with glucose and oxygen.

Since the known mammography method and apparatus is used for early detection of breast cancer, it concerns the screening of large numbers of people. Currently some 100 million women are being screened every year, consequently only very little time is available under screening circumstances. A typical routine screening will take some 5-10 minutes. Research shows that in screening programs a significant number of cancers are missed, which is due to the limited diagnostic value of the known mammography-test, and the related necessarily limited amount of time and facilities that are available for a screening. There is therefore a need to improve the mammography apparatus and method that are known from the prior art, such that the false negative rate is substantially decreased and that a more accurate detection of the possible occurrence of malignant cells becomes available so as to alleviate the subsequent second stage investigations for the occurrence of breast cancer.

Furthermore, an object of the invention is to devise the mammography-apparatus and method such that the above object is attained without compromising the current work-flow like the investigation rate with the known mammography-apparatus and method, and without complicating the current handling effort needed therefore.

US 2008/0249415 discloses a mammographic diagnosis apparatus that is based on X-ray imaging having a paddle that is provided with an opening portion which allows an ultrasonic probe to be fitted therein. The apparatus performs ultrasonic scanning on a breast compressed/fixed to the compression paddle, and reconstructs a compressed breast image by using the obtained ultrasonic image as a virtual image, thereby acquiring an ultrasonic image and volume data of the compressed/fixed breast. This known system is unsuitable for reliable screening operations on large numbers of people. The necessity to apply an opening portion in the compression paddle causes that the image data obtained with this known apparatus are unreliable in view of their lack of repeatability and technical considerations pertaining to the quality of the applied X-ray-imaging.

The article 'First Clinical Trials of the Twente Photoacoustic Mammoscope (PAM)', Proceedings of the SPIE - the International Society for Optical Engineering, 2007, ISSN-0277-786X, Vol. 6629, pages 66 2917-1-12 by Vaartjes S.E. et al. reports on the use of photoacoustics and the comparison of the resulting ultrasound images with earlier obtained X-ray mammograms. The article reports that the breast to be examined is to be suitably positioned between an illumination compartment and an ultrasound detector, in such a manner that the imaging will be performed principally in the area which harbours the tumour. The article reports further that compression of the breast is required to obtain a uniform thickness of the breast and a good acoustic contact with the detector. This is achieved by manually turning a handwheel of the compression mechanism until the patient indicates any discomfort. The person under examination is to remain immobile for a period up to 45 minutes during which period of time the scan will be performed. Ultrasound gel is used as a coupling medium. Evidently the technology disclosed in this article is aiming at imaging the detected abnormalities and is unsuited for purposes of screening large numbers of people in which the time available for any single screening will not be more than 5-10 minutes. Clearly also the article does not aim to provide a solution for screening, yet investigates the quality of photoacoustic imaging in comparison with X-ray imaging.

The general understanding in the art is that the application of photoacoustic spectroscopy such as disclosed in EP-A-1 493 380, in which the subject's tissue is irradiated with short duration pulses of light of a predetermined wavelength, requires the application of coupling gel to the detector elements for detecting an ultrasound image of the morphology of a human breast tissue that is excited with said short duration light pulses having wave lengths that lie in the absorption spectral brands of hemoglobine, to generate the intended photoacoustic signals.

**Http://en.wikipedia.org/wiki/medical ultrasonography** mentions for instance that sonographers typically use a handheld probe (called the transducer) that is placed directly on and moved over the patient. A water-based gel is used to couple the ultrasound between the transducer and patient. The state of the art is therefore that ultrasound requires the use of gels in order to allow that the ultrasound can be detected at all. The use of gel is however contra-indicative for use in an apparatus which is devised for executing a screening program for quickly detecting the occurrence of malignant cells in breasts.

Despite all contra-indicative teachings of the prior art the invention is embodied in a mammography-apparatus and method with the features of one or more of the appended claims. The mammography-apparatus of the invention is characterised in that it comprises a non-focussed laser-light source for pulsed laser-light, which during use is aimed at the breast, and that said apparatus comprises at least one non-contact ultrasound detector for contact-free detection of ultrasound originating from said breast so as to screen the occurrence of neo-angiogenesis.

The apparatus of the invention is neither intended nor suited for imaging of the photoacoustic signals with appropriate image quality, yet only intends to merely screen the occurrence of malignant cells in order to induce further investigation if the screening provides a positive result.

With the mammography-apparatus according to the invention it is possible to apply a method for executing a screening program to quickly detect the occurrence of malignant cells in a breast by using simultaneously and in combination an X-ray mammography detection method and a near-infrared contact-free photoacoustic detection method in which non-focussed pulsed laser-light is aimed at the breast and the induced ultrasound in the breast is measured with a detector and without applying a contact-gel between the detector and the breast. Contrary to all expectations this method and apparatus has proven effective for screening the occurrence of malignant cells and its feeding vessels in the breast.

It is possible that the laser-light source is located next or near to the X-ray source and that the laser-light-source points the laser-light through the paddle in the direction of the breast. Alternatively, glass fibre optics are comprised in the paddle for transmitting the laser-light such that said optic's outlet aim said laser-light towards the breast.

An aspect of the invention is that the laser-light is directionable and its direction measurable so as to monitor the direction of the laser-light when the ultrasound detector detects the laser-induced ultrasound. By varying the defocussing of the laser-light, specific segments of the breast can be excited. This offers the possibility that the direction of the defocussed pulsed laser-light is measured when the ultrasound induced in the breast is detected, and that said measured direction of the laser-light is used as a coarse measure for the direction at which malignant cells and their feeding vessels are located. Since the direction of the laser-light is known at the very moment an acoustic wave is generated, its direction can be matched with the spatial information of the mammogram. The mere existence of the acoustic signal is thus indicative for executing further mammographic detection but also for further ultrasound work-up, even when the mammography per se reveals no abnormalities.

A further aspect of the invention is that by using several non-contact ultrasound detectors a coarse localization of the abnormality that produces the acoustic signal can be made. For this purpose use is made of the different times of arrival of the said acoustic signal in the different non-contact ultrasound detectors. The times of arrival in the different non-contact ultrasound detectors are determined by the different distances travelled by the said ultrasound signal through the breast tissue and air on its way to the different detectors. Because of the synchronous x-ray imaging, this coarse localization can be easily related to this image.

The inventors point out that from the article Initial results of in vivo non-invasive cancer imaging in the human breast using near-infrared photoacoustics by Srirang Manohar et al., published 17 September 2007 in Volume 15, number 19 of Optics Express, pages 12277-12285, it is suggested to use near infrared optical imaging to obtain a 2D- or a 3D-image of the breast being investigated. There is, however, no suggestion or indication to depart from the complicated imaging method disclosed in this article (which needs the application of contact-gel) and to only use the photoacoustic effect known from pulsed laser-beam excitation as a general indicator for the occurrence of possibly malignant cells, which corresponds with the occurrence of specific physiological properties such as haemoglobin concentration and oxygen saturation. Due to this effect the apparatus and method of the invention is very well suited as a decision-tool for screening radiologists working on screening malignant cells of invasive breast tumours that characteristically show a microvascular network.

The invention will hereinafter be further elucidated with reference to a preferred embodiment of a mammography apparatus in accordance with the invention and with reference to the drawing.

In the drawing:
- Fig. 1 shows in a side view the mammography-apparatus of the invention;
- Fig. 2 shows schematically a first embodiment of the mammography-apparatus of the invention during screening of a breast,
- Fig. 3 shows a second embodiment of the mammography-apparatus of the invention when screening a breast for occurrence of malignant cells, and
- Fig. 4 shows a third embodiment of the mammography-apparatus of the invention during screening of a breast.

Same reference numerals applied in the figures refer to same parts.

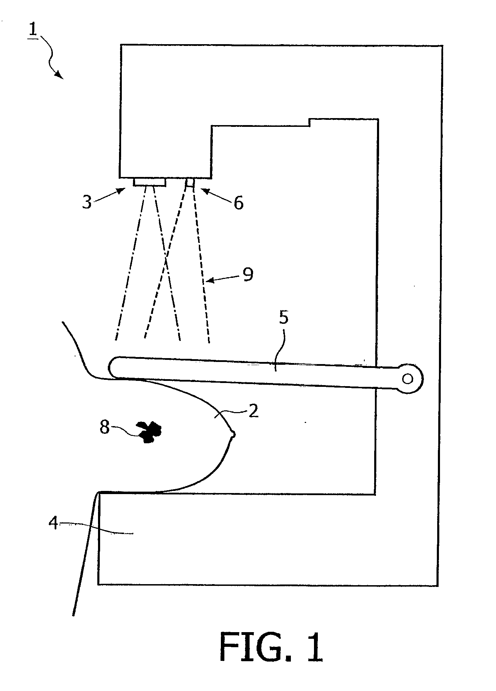

With reference first to Fig. 1 the mammography-apparatus 1 of the invention is shown for screening malignant cells 8 in a breast 2.

The mammography-apparatus 1 comprises an X-ray source 3, an X-ray detector 4 and a paddle 5 for pressing the breast 2 against said X-ray detector 4.

In accordance with the invention the mammography-apparatus 1 further comprises a laser-light-source 6 for transmitting pulsed defocussed laser-light 9 which during use is aimed at the breast 2 in a pre-determined direction which direction is monitored.

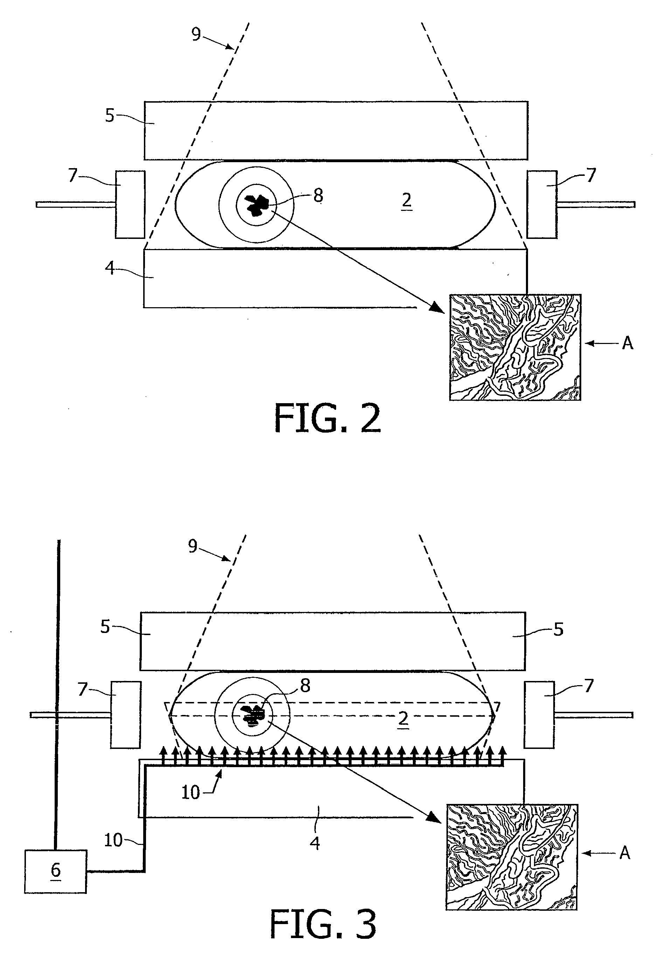

With reference to Fig. 2, 3 and 4 showing first, second and third embodiments of the apparatus 1 of the invention, it is shown that the apparatus 1 is also provided with at least one, and most commonly several non-contact ultrasound detectors 7 for detection of ultrasound that originates from said breast 2, which is induced by an excitation stemming from the said defocussed laser-light 9. Thus, it is possible to generally screen the occurrence of malignant cells 8, which are shown in the detailed view A of Fig. 2, 3 and 4 to be characterized by a dense microvascular network. It is important to note that the detectors 7 are non-contact detectors and that their operation is without application of any contact-gel between the detectors 7 and the breast 2.

The acoustic effect is generated by the pulsed laser-light in the microvascular haemoglobin containing network that is typical for neoplasia like malignant invasive tumours, and is not induced by pseudo-malignant lesions which can also be found in mammography. This aspect makes it possible that many unnecessary referrals of screened women for further assessment are avoided which would otherwise cause a lot of anxiety and diagnostic work-up, like invasive punctures and even diagnostic operations.

To allow detection of the position at which the malignant cells 8 are located, the laser-light 9 may be directionable and its direction measurable so as to monitor the direction of the laser-light 9 when the ultra-sound detector or detectors 7 detect the laser-induced ultrasound. Thus a coarse measure is provided for the direction at which malignant cells 8 and their feeding vessels are located.

A further aspect of the invention is that by using several non-contact ultrasound detectors a coarse localization of the abnormality that produces the acoustic signal can be made. For this purpose use is made of the different times of arrival of the said acoustic signal in the different non-contact ultrasound detectors. The times of arrival in the different non-contact ultrasound detectors are determined by the different distances travelled by the said ultrasound signal through the breast tissue and air on its way to the different detectors. Because of the synchronous x-ray imaging, this coarse localization can be easily related to this image.

Fig. 2 shows that the laser-light 9 that originates from the laser-light source 6 as shown in Fig. 1 is able to transmit through the paddle 5 and induce ultrasound pulses in the breast 2. The laser-light source 6 used is for instance typically having an energy of 30 mJ/cm² operating at 5 nanoseconds pulse width with a 10 Hertz repetition rate. The laser-light source 6 is transmitting in the 1064 nm range.

Fig. 3 shows a variation to the embodiment shown in Fig. 2 in that apart from the laser-light that is directed through the paddle 5 from above, also glass fibre optics 10 are provided in the housing of the X-ray detector 4, that transmit light-pulses from the laser-light-source 6 at appropriate locations from below into the breast 2.

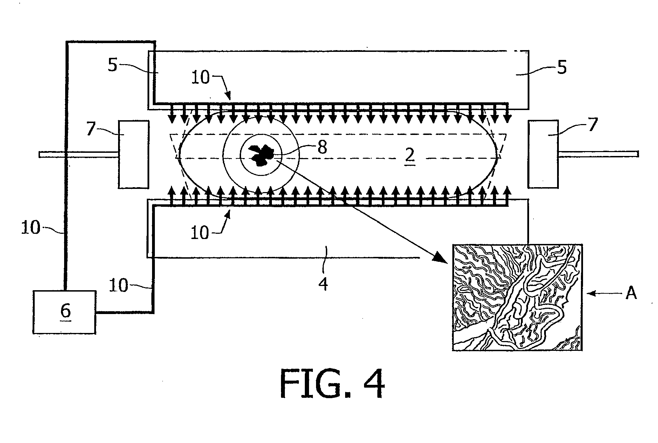

Fig. 4 shows still a further variation to the embodiments shown in Fig. 2 and 3. In this embodiment there is no laser-light transmitted through the paddle 5, yet there are at appropriate locations glass fibre optics 10 provided in the paddle 5 that transmit the light pulses from the laser-light source 6 from above into the breast 2, in addition to light pulses through the glass fibre optics 10 in the housing of the x-ray detector 4, coming from below as shown in Fig. 3.

The embodiments shown in Figures 3 and 4 are preferred embodiments and have improved ultrasound response to possible occurrences of malignant cells as compared to the embodiment shown in Fig. 2 which only induces ultrasound with light pulses that are introduced into the breast from above.

The use of the photoacoustic effect as elucidated hereabove is particularly well suited to compensate the less effective screening results that come available with the known mammography-apparatus. In combination, the screening results are effectively improved. Both a significantly lower false negative rate and a lower false positive rate as compared to the known mammography method and apparatus results, without imparting any need to lengthen or complicate the screening-method of the prior art.

## Claims

1. Mammography-apparatus (1) for detecting malignant cells (8) in a breast (2) comprising an X-ray source (3), an X-ray detector (4) and a paddle (5) for pressing the breast (2) against said X-ray detector (4), **characterized in that** the mammography-apparatus further comprises a non-focussed laser-beam source (6) for pulsed laser-light, which during use is aimed at the breast (2), and said apparatus comprises at least one non-contact ultrasound detector (7) for contact-free detection of an ultrasound originating from said breast (2) so as to screen the occurrence or non-occurrence of said malignant cells (8).

2. Mammography-apparatus (1) according to claim 1, **characterized in that** glass fibre optics (10) are comprised in the paddle (5) for transmitting the laser-light, such that said optic's outlet aim said laser-light towards the breast (2).

3. Mammography-apparatus according to claim 1 or 2, **characterized in that** glass fibre optics (10) are comprised in a housing of the X-ray detector (4) for transmitting the laser-light, such that said optic's outlet aim said laser-light towards the breast (2).

4. Mammography-apparatus according to any one of claims 1-3, **characterized in that** the laser-light is directionable and its direction measurable so as to monitor the direction of the laser-light when the non-contact ultrasound detector (7) detects the laser-induced ultrasound.

5. Method for screening the occurrence of malignant cells (8) in a breast (2) by using simultaneously and in combination an X-ray mammography detection method and a near-infrared photoacoustic contact-free detection method in which non-focussed pulsed laser-light (9) is aimed at the breast (2) and the induced ultrasound in the breast (2) is measured with a detector (7) and without applying a contact-gel between the detector and the breast (2), so as to screen the occurrence or non-occurrence of said malignant cells (8) and their feeding vessels in the breast (2).

6. Method according to claim 5, **characterized in that** a plurality of detectors (7) are used for contact-free detection of ultrasound induced in the breast (2) and that for each single detector (7) a time of arrival of said ultrasound at the concerning detector is measured, and that all times of arrival of said ultrasound at the respective detectors (7) are used to estimate the location of the malignant cells (8).

7. Method according to claim 5 or 6, wherein the direction of the pulsed laser-light (9) is detected when the ultrasound induced in the breast (2) is measured, and that said detected direction of the laser-light (9) is used as a measure for the direction at which malignant cells (8) and their feeding vessels are located.

## Patentansprüche

1. Mammographiegerät (1) zum Detektieren maligner Zellen (8) in einer Brust (2), umfassend eine Röntgenstrahlenquelle (3), einen Röntgendetektor (4) und eine Platte (5) zum Pressen der Brust (2) gegen den Röntgendetektor (4), **dadurch gekennzeichnet, dass** das Mammographiegerät ferner eine nicht-fokussierte Laserstrahlquelle (6) für gepulstes Laserlicht umfasst, das während der Verwendung auf die Brust (2) gerichtet wird, und wobei das Gerät mindestens einen kontaktlosen Ultraschalldetektor (7) zur kontaktfreien Detektion eines Ultraschalls, der in der Brust (2) gebildet wird, umfasst, so dass das Auftreten oder Nichtauftreten der malignen Zellen (8) untersucht wird.

2. Mammographiegerät (1) nach Anspruch 1, **dadurch gekennzeichnet, dass** Glasfaseroptik (10) in der Platte (5) für die Übertragung des Laserlichtes derart enthalten ist, dass der Ausgang der Optik das Laserlicht in Richtung der Brust (2) richtet.

3. Mammographiegerät nach Anspruch 1 oder 2, **dadurch gekennzeichnet, dass** Glasfaseroptik (10) in einem Gehäuse des Röntgendetektors (4) für die Übertragung des Laserlichtes derart enthalten ist, dass der Ausgang der Optik das Laserlicht in Richtung der Brust (2) richtet.

4. Mammographiegerät nach einem der Ansprüche 1 - 3, **dadurch gekennzeichnet, dass** das Laserlicht ausgerichtet werden kann und seine Richtung messbar ist, so dass die Richtung des Laserlichtes überwacht wird, wenn der kontaktlose Ultraschalldetektor (7) den laserinduzierten Ultraschall detektiert.

5. Verfahren zum Untersuchen des Auftretens maligner Zellen (8) in einer Brust (2) durch gleichzeitige und kombinierte Verwendung eines Röntgenmammographiedetektionsverfahrens und eines photoakustischen kontaktfreien Detektionsverfahrens für den nahen Infrarotbereich, bei dem nichtfokussiertes gepulstes Laserlicht (9) auf die Brust (2) gerichtet und der induzierte Ultraschall in der Brust (2) mit einem Detektor (7) und ohne Aufbringen eines Kontaktgels zwischen dem Detektor und der Brust (2) gemessen wird, so dass das Auftreten oder Nichtauftreten der malignen Zellen (8) und von deren Versorgungsgefäßen in der Brust (2) untersucht wird.

6. Verfahren nach Anspruch 5, **dadurch gekennzeichnet, dass** eine Vielzahl von Detektoren (7) für die kontaktfreie Detektion von Ultraschall, der in der Brust (2) induziert wird, verwendet wird, und dass für jeden einzelnen Detektor (7) eine Ankunftszeit des Ultraschalls an dem betreffenden Detektor gemessen wird, und dass alle Ankunftszeiten des Ultraschalls an den jeweiligen Detektoren (7) zur Bestimmung der Lage der malignen Zellen (8) verwendet werden.

7. Verfahren nach Anspruch 5 oder 6, wobei die Richtung des gepulsten Laserlichtes (9) detektiert wird, wenn der Ultraschall, der in der Brust (2) induziert wird, gemessen wird, und die detektierte Richtung des Laserlichtes (9) als ein Maß für die Richtung verwendet wird, in der sich maligne Zellen (8) und deren Versorgungsgefäße befinden.

## Revendications

1. Appareil de mammographie (1) pour déceler des cellules malignes (8) dans un sein (2), comprenant une source de rayons X (3), un détecteur de rayons X (4) et une plaque (5) pour comprimer le sein (2) contre ledit détecteur de rayons X (4), **caractérisé en ce que** l'appareil de mammographie comprend, en outre, une source de faisceau laser non focalisé (6) pour lumière laser pulsée, qui, à l'utilisation, vise le sein (2), et ledit appareil comprend au moins un détecteur d'ultrasons sans contact (7) pour détecter, sans contact, un ultrason provenant dudit sein (2) afin de dépister la présence ou l'absence de dites cellules malignes (8).

2. Appareil de mammographie (1) selon la revendication 1, **caractérisé en ce que** des fibres de verre optiques (10) sont incluses dans la plaque (5) pour transmettre la lumière laser, de manière que lesdites sorties optiques dirigent ladite lumière laser vers le sein (2).

3. Appareil de mammographie (1) selon la revendication 1 ou 2, **caractérisé en ce que** des fibres de verre optiques (10) sont incluses dans un logement du détecteur de rayons X (4) pour transmettre la lumière laser, de manière que lesdites sorties optiques dirigent ladite lumière laser vers le sein (2).

4. Appareil de mammographie (1) selon l'une quelconque des revendications 1 à 3, **caractérisé en ce que** la lumière laser est dirigeable et sa direction est mesurable afin de contrôler la direction de la lumière laser lorsque le détecteur d'ultrasons sans contact (7) détecte l'ultrason induit par laser.

5. Procédé pour dépister la présence de cellules malignes (8) dans un sein (2) par utilisation, simultanément et en combinaison, d'une méthode de détection par mammographie par rayons X et d'une méthode de détection photo acoustique sans contact dans le proche infrarouge dans laquelle une lumière laser pulsée (9) non focalisée vise le sein (2) et l'ultrason induit dans le sein (2) est mesuré au moyen d'un détecteur (7) et sans appliquer de gel de contact entre le détecteur et le sein (2), afin de dépister la présence ou l'absence de dites cellules malignes (8) et de leurs vaisseaux nourriciers dans le sein (2).

6. Procédé selon la revendication 5, **caractérisé en ce qu'**une pluralité de détecteurs (7) sont utilisés pour la détection sans contact d'ultrason induit dans le sein (2) est **en ce que**, pour chaque détecteur unique (7), un temps d'arrivée dudit ultrason au détecteur concerné est mesuré, et **en ce que** tous les temps d'arrivée dudit ultrason aux détecteurs (7) respectifs sont utilisés pour estimer l'emplacement des cellules malignes (8).

7. Procédé selon la revendication 5 ou 6, dans lequel la direction de la lumière laser pulsée (9) est détectée lorsque l'ultrason induit dans le sein (2) est mesuré, et ladite direction détectée de la lumière laser (9) est utilisée comme mesure pour la direction dans laquelle les cellules malignes (8) et leurs vaisseaux nourriciers sont situés.
